# EUROPÄISCHE PATENTSCHRIFT

(11) **EP 3 318 569 B1**
(45) Veröffentlichungstag und Bekanntmachung des Hinweises auf die Patenterteilung: **17.07.2019**
(21) Anmeldenummer: 16197715.2
(22) Anmeldetag: 08.11.2016
(51) Int. Cl.: C07F 9/145, C07F 15/00, C07C 45/50

(54) **BISPHOSPHITE MIT 2,4-TERT.-BUTYLPHENYL-EINHEITEN UND DEREN VERWENDUNG ALS LIGANDEN IN DER HYDROFORMYLIERUNG**
BIS-PHOSPHITES WITH 2,4-TERT. BUTYLPHENYL UNITS AND THEIR USE AS LIGANDS IN THE HYDROFORMYLATION
BISPHOSPHATE PRÉSENTANT DES UNITÉS DE 2,4-TERT.-BUTYLPÉNYL ET SON UTILISATION EN TANT QUE LIGANDS DANS L'HYDROFORMYLATION

(43) Veröffentlichungstag der Anmeldung: 09.05.2018
(73) Patentinhaber: Evonik Degussa GmbH, 45128 Essen (DE)
(72) Erfinder: DYBALLA, Katrin Marie, 45657 Recklinghausen (DE); FRANKE, Robert, 45772 Marl (DE); FRIDAG, Dirk, 45721 Haltern am See (DE); HESS, Dieter, 45770 Marl (DE)

(56) Entgegenhaltungen:
- EP-A2- 0 518 241
- WO-A1-95/18089
- WO-A1-96/11182
- WO-A1-99/38832

## Beschreibung

Die Erfindung betrifft Bisphosphite mit 2,4-*tert*.-Butylphenyl-Einheiten und ein Verfahren zu deren Herstellung. Des Weiteren betrifft die Erfindung die Verwendung der Verbindungen als Liganden in einem Ligand-Metall-Komplex. Die Verbindung, wie auch der Komplex, können als katalytisch aktive Zusammensetzung in Hydroformylierungsreaktionen eingesetzt werden.

Phosphorhaltige Verbindungen spielen als Liganden in einer Vielzahl von Reaktionen eine entscheidende Rolle. Hierzu zählen Phosphitliganden, also Verbindungen, die P-O-Bindungen enthalten, die in der Hydrierung, Hydrocyanierung und vor allem in der Hydroformylierung Anwendung finden.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor P^{III}. Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803 EP 0518241 A2 betrifft ein Hydroformylierungsverfahren einer Olefinverbindung, bei der ein modifizierter Katalysator eines Metalls der Gruppe VIII verwendet wird.

Literaturbekannt ist die Synthese symmetrisch aufgebauter Bisphosphite, wie sie beispielsweise in US 4,769,498 offenbart wurden, und deren Verwendung in katalytisch aktiven, übergangsmetallhaltigen Zusammensetzungen zur Hydroformylierung ungesättigter Verbindungen.

In US 4,769,498, wie auch in US 5,723,641 werden bevorzugt symmetrisch aufgebaute Bisphosphite hergestellt und als Liganden zur Hydroformylierung verwendet. Die in der Hydroformylierung verwendeten symmetrisch aufgebauten Bisphosphitliganden werden bei tiefen Temperaturen hergestellt. Die Einhaltung dieser tiefen Temperaturen ist zwingend erforderlich, da höhere Temperaturen gemäß dieser US-Schriften zu Umlagerungen und letztlich zu unsymmetrisch aufgebauten Bisphosphiten führen würden.

Ein Ligand, welcher eine sehr gute Ausbeute liefert, ist der Ligand gemäß Formel (**2**):

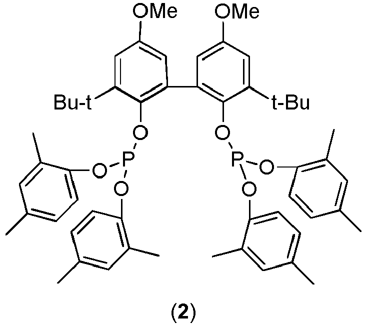

Bei der Hydroformylierung von Raffinat 3 liefert der Ligand (**2**) allerdings fast ausschließlich (zu 99%) das n-Pentanal. Raffinat 3 ist ein Gemisch aus: ca. 26% 1-Buten, 32% trans-2-Buten, 17% cis-2-Buten, 25% n-Butan und Spuren an Iso-Butan und Neo-Pentan.

Für manche technischen Anwendungen ist es aber erstrebenswert, dass das Verhältnis von Linearem Aldehyd (= n-Aldehyd) zu verzweigtem Aldehyd (= iso-Aldehyd) möglichst ausgeglichen ist.

### Definition der Selektivität:

Bei der Hydroformylierung gibt es die n/iso-Selektivitäte: das Verhältnis von linearem Aldehyd (= n) zu verzweigtem Aldehyd (= iso). Hierbei bedeutet die Regioselektivität bezüglich des n-Aldehyden, dass diese Menge an linearem Produkt gebildet wurde. Die restlichen Prozente entsprechen dann dem verzweigten Isomer. Bei einer Regioselektivität von 50% entstehen also n-Aldehyd und iso-Aldehyd zu gleichen Teilen.

Die technische Aufgabe der Erfindung ist die Bereitstellung eines neuen Liganden, welcher in der Hydroformylierung von ungesättigten Verbindungen nicht die aus dem Stand der Technik zuvor aufgezeigten Nachteile, sondern die folgenden Eigenschaften aufweist:
1) eine gute Aktivität/Ausbeute,
2) eine n-Regioseliktivität in Bezug auf die Hydroformylierung von 50% +/- 15%.

Die Aufgabe wird gelöst durch eine Verbindung gemäß Anspruch 1.

### Verbindung der Formel (I):

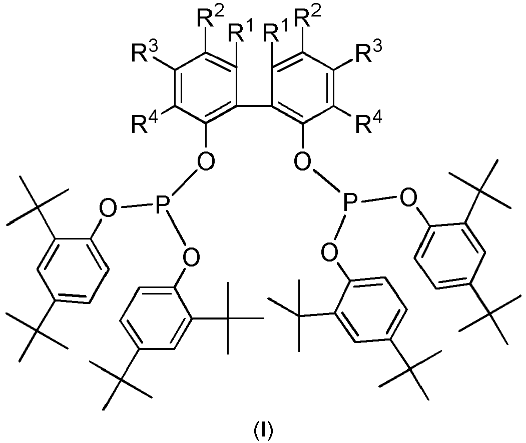

wobei
R¹, R², R³, R⁴ ausgewählt sind aus: -H, -(C₁-C₁₂)-Alkyl, -O-(C₁-C₁₂)-Alkyl,
wobei die genannten Alkylgruppen wie folgt substituiert sein können:
   substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen; die Substituenten sind unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, wobei mindestens einer der Reste R¹,R²,R³,R⁴ nicht für -H steht.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt -(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Die Erläuterungen zum Ausdruck -(C₁-C₁₂)-Alkyl gelten auch für die Alkylgruppen in -O-(C₁-C₁₂)-Alkyl, also in -(C₁-C₁₂)-Alkoxy. Vorzugsweise handelt es sich dabei um unsubstituierte geradkettige oder verzweigte -(C₁-C₆)-Alkoxygruppen.

Substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen; die Substituenten sind unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

In einer Ausführungsform sind R² und R⁴ ausgewählt aus: -(C₁-C₁₂)-Alkyl, -O-(C₁-C₁₂)-Alkyl.

In einer Ausführungsform steht R² für -O-(C₁-C₁₂)-Alkyl.

In einer Ausführungsform steht R⁴ für -(C₁-C₁₂)-Alkyl.

In einer Ausführungsform sind R¹, R², R³, R⁴ ausgewählt aus: -H, -Me, -tBu, -OMe, -iPr.

In einer Ausführungsform steht R¹ für -H.

In einer Ausführungsform sind R², R⁴ ausgewählt aus: -Me, -tBu, -OMe, -iPr.

In einer Ausführungsform sind R², R⁴ ausgewählt aus: -Me, -tBu, -OMe,

In einer Ausführungsform ist R² ausgewählt aus: -Me, -tBu, -OMe.

In einer Ausführungsform ist R² ausgewählt aus: -tBu, -OMe.

In einer Ausführungsform ist R² ausgewählt aus: -Me, -OMe.

In einer Ausführungsform steht R² für -OMe.

In einer Ausführungsform steht R³ für -H.

In einer Ausführungsform ist R⁴ ausgewählt aus: -Me, -tBu, -OMe.

In einer Ausführungsform ist R⁴ ausgewählt aus: -tBu, -OMe.

In einer Ausführungsform ist R⁴ ausgewählt aus: -Me, -tBu.

In einer Ausführungsform steht R⁴ für -tBu.

In einer Ausführungsform weist die Verbindung die Formel (**1**) auf:

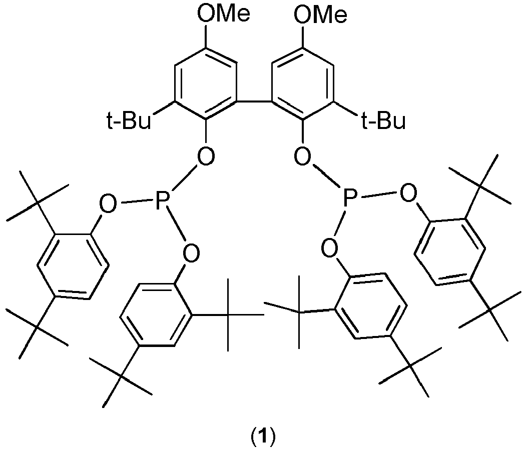

Neben den Verbindungen werden auch Komplexe beansprucht, welche die Verbindung umfassen.

### Komplex gemäß der Formel (II):

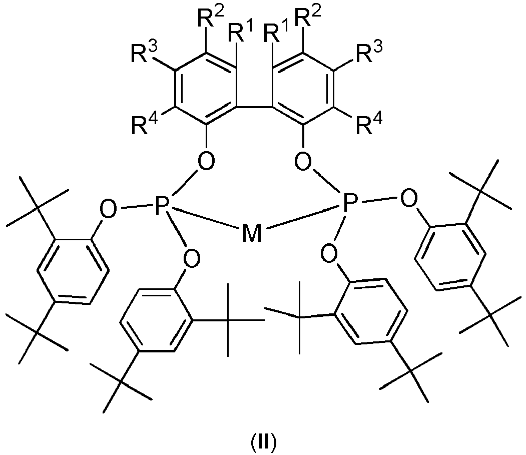

wobei
R¹, R², R³, R⁴ ausgewählt sind aus: -H, -(C₁-C₁₂)-Alkyl, -O-(C₁-C₁₂)-Alkyl,
wobei die genannten Alkylgruppen wie folgt substituiert sein können:
   substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen; die Substituenten sind unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl;
   und M ausgewählt ist aus: Rh, Ru, Co, Ir.

In einer bevorzugten Ausführungsform ist M = Rh.

Die in Zusammenhang mit der Formel (**I**) oben angeführten Ausführungsformen und Auswahlmöglichkeiten für die Reste R¹, R², R³, R⁴ gelten analog für die Formel (**II**).

### Komplex gemäß der Formel (III):

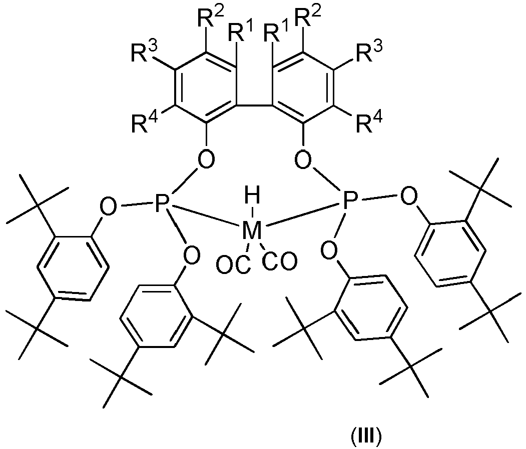

wobei
R¹, R², R³, R⁴ ausgewählt sind aus: -H, -(C₁-C₁₂)-Alkyl, -O-(C₁-C₁₂)-Alkyl,
wobei die genannten Alkylgruppen wie folgt substituiert sein können:
   substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen; die Substituenten sind unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl;
   und M ausgewählt ist aus: Rh, Ru, Co, Ir.

In einer bevorzugten Ausführungsform ist M = Rh.

Die in Zusammenhang mit der Formel (**I**) oben angeführten Ausführungsformen und Auswahlmöglichkeiten für die Reste R¹, R², R³, R⁴ gelten analog für die Formel (**III**).

Neben der Verbindung und den Komplexen, welche die Verbindung umfassen, wird auch die Verwendung der Verbindung (**I**) beziehungsweise der Komplexe (**II**) oder (**III**) zur Katalyse einer Hydroformylierungsreaktion beansprucht.

Verwendung der Verbindung (**I**) in einem Ligand-Metall-Komplex zur Katalyse einer Hydroformylierungsreaktion.

Verwendung des Komplexes (**II**) zur Katalyse einer Hydroformylierungsreaktion.

Verwendung des Komplexes (**III**) zur Katalyse einer Hydroformylierungsreaktion.

Des Weiteren wird auch das Hydroformylierungsverfahren beansprucht, in welchem die Verbindung (**I**) beziehungsweise der Komplexe (**II**) oder (**III**) zum Einsatz kommt.

Verfahren umfassend die Verfahrensschritte:
a) Vorlegen eines Olefins,
b) Zugabe eines zuvor beschriebenen Komplexes,
   oder einer zuvor beschriebenen Verbindung und einer Substanz, welche ein Metall ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

Die Verfahrensschritte a) bis c) können hierbei in beliebiger Reihenfolge erfolgen.

Die Verbindung kann als Ligand in einem Ligand-Metall-Komplex eingesetzt werden.

Es kann hierbei auch ein Überschuss an Liganden verwendet werden und nicht zwangsläufig jeder Ligand liegt gebunden in Form eines Ligand-Metall-Komplexes vor, sondern ist als freier Ligand im Reaktionsgemisch enthalten.

Die Reaktion wird bei üblichen Bedingungen durchgeführt.

Bevorzugt sind eine Temperatur von 80 °C bis 160 °C und ein Druck von 1 bis 300 bar. Besonders bevorzugt sind eine Temperatur von 100 °C bis 160 °C und ein Druck von 15 bis 250 bar.

In einer bevorzugten Ausführungsform ist das Metall Rh.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1-Buten, 2-Buten, 1- oder 2-Penten, 2-Methyl-1-buten, 2-Methyl-2-buten, 3-Methyl-1-buten, 1-, 2- oder 3,-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Di-n-buten, Di-iso-buten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung von Propen oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabuten) sowie durch Cooligomerisierung von Olefinen mit unterschiedlicher Anzahl von Kohlenstoffatomen (bevorzugt 2 bis 4) hergestellte Olefingemische.

Mit dem erfindungsgemäßen Verfahren können unter Verwendung der erfindungsgemäßen Liganden α-Olefine, endständig verzweigte, innenständige und innenständig verzweigte Olefine hydroformyliert werden.

Im Folgenden soll die Erfindung anhand von Ausführungsbeispielen näher erläutert werden.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Phosphortrichlorid (Aldrich) wurde vor dem Einsatz unter Argon destilliert. Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR31P = SR_{1H} ^{∗} (BF_{31P} / BF_{1H}) = SR_{1H} ^{∗} 0,4048. (Robin K. Harris, Edwin D. Becker, Sonia M. Cabral de Menezes, Robin Goodfellow, and Pierre Granger, Pure Appl. Chem., 2001, 73, 1795-1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84).

Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

### Allgemeine Reaktionsgleichung (Vergleichsligand)

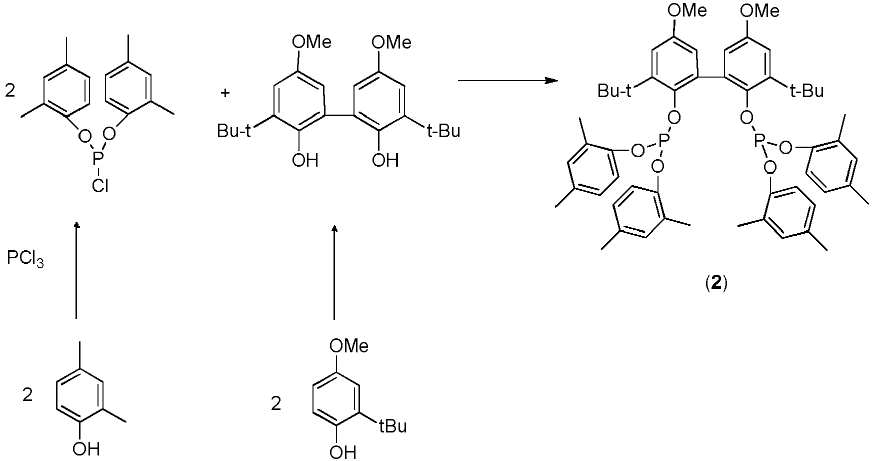

### Herstellung von Bis-(2,4-dimethylphenyl)-chlorophosphit

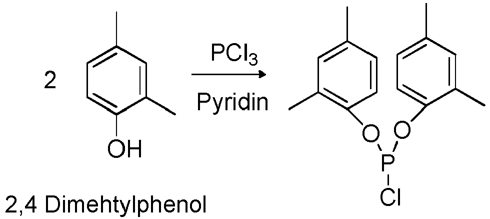

In einem sekurierten 1200 ml Glasreaktor, mit Tropftrichter versehen, wurden 50 g PCl₃ (0.363 mol) und 86 g Pyridin (1.076 mol) in 380 ml getrocknetem Toluol vorgelegt. Die milchig-gelbe PCl₃/Pyridin-Lösung wurde unter Rühren auf -7 °C heruntergekühlt. Dann wurde in den Tropftrichter 86 ml 2,4-Dimethylphenol (0.720 mol) hinzugegeben und in 380 ml getrocknetem Toluol gelöst. Zur Durchführung wurde die Phenol/Toluol-Lösung langsam und stetig zur PCl₃/Pyridin-Lösung hinzugetropft. Über Nacht wurde die Reaktionsmischung unter Rühren auf Raumtemperatur gebracht.

Zur Aufarbeitung wurde das entstandene Hydrochlorid abfiltriert und mit 60 ml getrocknetem Toluol nachgespült und die entstandene Mutterlauge unter vermindertem Druck bis zur Trockne eingeengt.

Zur weiteren Aufarbeitung wurde die Rohlösung destilliert. Hierfür wurde ein Spitzkolben mit der Rohlösung befüllt, darauf kam eine kurze Destillationsbrücke ohne Kühlmantel. An der oberen Öffnung wurde das Thermometer platziert, am anderen Ende wurde eine Spinne mit vier weiteren Spitzkolben befestigt. Anschließend wurde diese Apparatur mit einer Kühlfalle verbunden und von dort aus mit der Hochvakuumpumpe. Der Spitzkolben mit dem zu destillierenden Rohliganden wurde mittels Ölbad erwärmt. Zuerst wurde der Vorlauf bei einer Kopftemperatur von 25-30 °C abgenommen. Anschließend wurde die Spinne weiter gedreht und bei einer Kopftemperatur von 140 °C wurde der Hauptlauf abgenommen. Als keine Tropfen mehr im Hauptlauf ankamen, wurde die Destillation abgestellt, die Pumpe runtergefahren und der Hauptlauf in dem entsprechenden Spitzkolben entnommen, verschlossen und analysiert.

### Ergebnis:

Gesamtmasse: 56.7 g (46% Ausbeute)

### Herstellung von 3,3'-di-tert-butyl-5,5'-dimethoxy-[1,1'-biphenyl]-2,2'-diyltetrakis(2,4-dimethylphenyl)bis(phosphite)

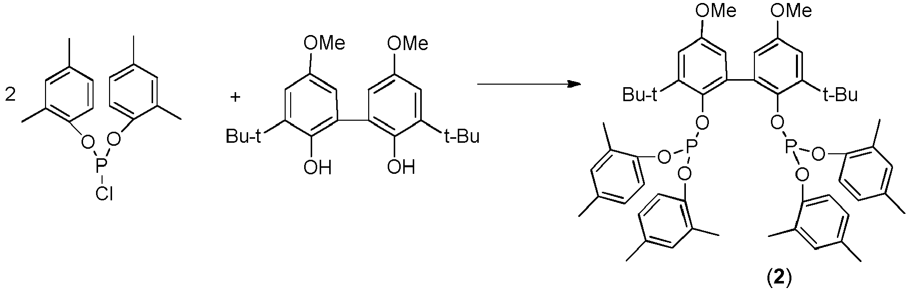

In einem 1000 mL Schlenkkolben wurden zu 51,86 g (0,153 mol) Bis-(2,4-dimethylphenyl)-chlorophosphit unter Rühren bei Raumtemperatur 260 ml getrocknetem Acetonitril zugegeben und das Chlorophosphit aufgelöst.

In einen zweiten 250 ml Schlenkkolben wurden 20,1 g (0,056 mol) 3,3'-Di-tert.-butyl-5,5'-dimethoxy-[1,1'-biphenyl]-2,2'diol mit 12,4 ml (0,153 mol) Pyridin und 155 ml getrocknetem Acetonitril versetzt. Nun wurde die Chlorophosphitlösung im Schlenkkolben auf 0 °C abgekühlt. Anschließend wurde langsam unter kräftigem Rühren die Biphenol/Pyridin-Lösung zugetropft. Die Reaktionsmischung wurde ca. 3 h bei dieser Temperatur gehalten und dann ganz langsam über Nacht auf Raumtemperatur gebracht.

Dann wurde die Suspension abfiltriert, mit 30 ml Acetonitril gut nachgewaschen und getrocknet.

### Ergebnis:

Masse: 44,01 g (Ausbeute: 85%)

### Chlorreduzierung

Um in diesem Rohliganden den Chlorgehalt zu senken wurde dieser aufgereinigt.

Die angegebenen Chlorgehalte verstehen sich als Gesamtchlorgehalte.

Der Gesamtchlorgehalt wird nach Wickbold bestimmt: Probenvorbereitung nach DIN 51408 und Messung per lonenchromatographie nach DIN EN ISO 10304.

Es wurden 5,15 g 3,3'-di-tert-butyl-5,5'-dimethoxy-[1,1'-biphenyl]-2,2'-diyltetrakis(2,4-dimethylphenyl)bis(phosphite) in einen 250 ml Schlenkkolben mit 15 ml entgastem Toluol und 5 ml Pyridin bei 100 °C zum Rühren gebracht bis alles aufgelöst war. Nachdem alles gelöst war wurde noch für weitere 15 min die Temperatur gehalten und dann auf 90 °C abgekühlt.

In der Zwischenzeit wurde in einen weiteren 250 ml Schlenkkolben 100 ml Heptan und 5ml Pyridin gegeben und die Lösung wurde auf 0 °C herunter gekühlt. Anschließend wurde die Lösung mit dem 3,3'-di-tert-butyl-5,5'-dimethoxy-[1,1'-biphenyl]-2,2'-diyltetrakis(2,4-dimethylphenyl)bis(phosphite) über die Fritte in die kalte Heptan/Pyridin Lösung gegeben und für 3 h bei 0 °C gerührt. Auch hierbei fiel nichts aus. Also wurde auch hier mittels Vakuumpumpe das Lösemittel abgezogen bis der Feststoff ausgefallen und getrocknet war. Anschließend wurden auf den getrockneten Feststoff 50 ml Acetonitril gegeben. Diese Suspension wurde über zwei Tage bei Raumtemperatur gerührt, abgefrittet und mittels Vakuumpumpe getrocknet.

### Ergebnis:

Masse: 3,7 g
Chlorbestimmung: 20/20 ppm

### Allgemeine Reaktionsgleichung (erfindungsgemäßer Ligand)

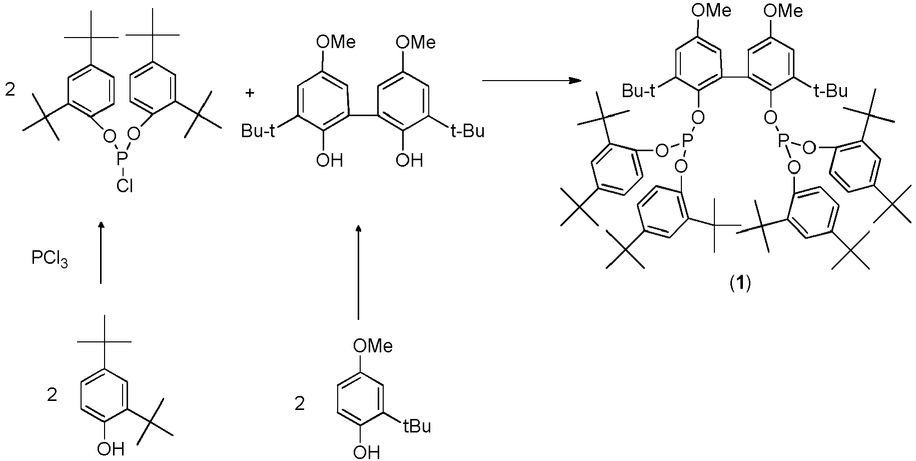

### Herstellung von Bis-(2,4-tert.-butylphenyl)-chlorophosphit

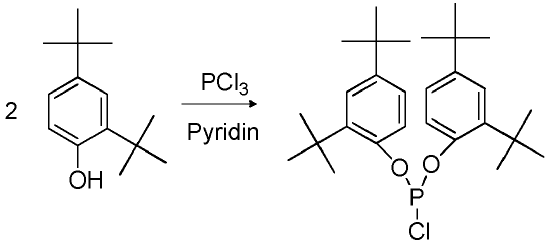

In einem sekurierten 2000 ml Schlenkkolben, mit Trofptrichter versehen, wurden 240 ml getrocknetes Toluol und 8,8 ml Phosphortrichlorid vorgelegt und unter Rühren auf 0 °C abgekühlt.

In einem zweiten 1000 ml Schlenkkolben wurden 41,6 g 2,4-Di-tert.-Butylphenol abgewogen, in 415 ml getrocknetem Toluol gelöst und mit 100 ml Triethylamin versetzt.

Dann wurde die Phenol/Triethylamin-Lösung in den Tropftrichter überführt. Nun wurde unter kräftigem Rühren innerhalb von 4h die Phenol/Triethylamin-Lösung zur gut gekühlten Toluol/Phosphortrichlorid-Lösung getropft. Dabei wurde auf eine konstante Tropfgeschwindigkeit von einem Tropfen pro Sekunde geachtet. Anschließend wurde die Mischung auf Raumtemperatur erwärmt und 12 Stunden gerührt, danach für 4 Stunden auf 45 °C erwärmt.

Zur Aufarbeitung wurde das entstandene Triehtylaminhydrochlorid abfiltriert und mit 20 ml getrocknetem Toluol nachgewaschen, das Filtrat wurde unter vermindertem Druck bis zur Trockene eingeengt.

### Ergebnis:

Masse: 41,5 g (Ausbeute 82%)

### Herstellung von 3,3'-di-tert-butyl-5,5'-dimethoxy-[1,1'-biphenyl]-2,2'-diyl-tetrakis(2,4-di-tert-butylphenyl)bis(phosphite)

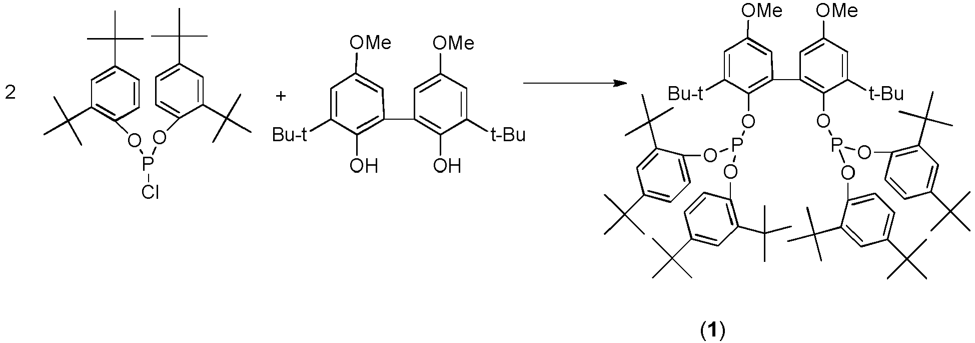

12.0 g Bi-(2,4-di-tert.-butylphenyl)-chlorophopshit (0.021 mol) wurden in einem 250 ml Schlenkkolben in 100 ml getrocknetem Acetonitril gelöst.

In einen zweiten Schlenkkolben (250 ml) wurden 3,2 g (0,009 mol) 3,3'-Di-tert.-butyl-5,5'-dimethoxy-[1,1'-biphenyl]-2,2'diol unter Rühren zu 50 ml getr. Acetonitril und 2,2 g entgastes N,N-Dimethylaminobutan (DMAB) gegeben. Dabei entstand eine Suspension.

Dann wurde innerhalb einer 1 h die Biphenol/DMAB-Lösung bei 0 °C zur Chlorophosphitlösung getropft. Anschließend wurde diese über Nacht auf Raumtemperatur erwärmt und dann für 12 Stunden bei 55 °C gerührt. Anschließend wurde die Reaktionslösung wieder auf Raumtemperatur abgekühlt und abfiltriert. Der Feststoff wurde zweimal mit je 20 ml getrocknetem Acetonitril gewaschen und dann unter vermindertem Druck getrocknet und analysiert.

Um das Produkt vom Tris(2,4-di-tert-butylphenyl)phosphit zu trennen, wurde der Feststoff von der Fritte in einem 500 ml Schlenkkolben umgefüllt, und mit 400 ml getrocknetem Acetonitril versetzt. Anschließend wurde der Schlenkkolben für 5 h auf 60 °C erwärmt, heiß filtriert und einmal mit 20 ml getrocknetem Acetonitril nachgewaschen.

Das Filtrat wurde unter vermindertem Druck eingeengt und analysiert.

Um das Produkt weiter aufzureinigen, wurde der Schlenkkolben mit 80 ml getrocknetem Acetonitril versetzt und die Suspension wurde für 1,5 h auf 60 °C erwärmt und dann heiß gefrittet. Danach wurde das Filtrat unter vermindertem Druck eingeengt.

Um das Amiohydrochlorid zu entfernen, wurde der Feststoff in 20 ml getrocknetem Toluol gelöst, 1/2 h bei Raumtemperatur gerührt und anschließend filtriert. Der Feststoff wurde 2 mal mit 10 ml getrocknetem Toluol nachgewaschen. Anschließend das Filtrat mittels vermindertem Druck eingeengt und analysiert.

### Ergebnis:

Masse: 3.2 g (26.7 % Ausbeute)

### Durchführung der Katalyseversuche

### Versuchsbeschreibung - allgemein

In einem 100 ml-Autoklaven der Fa. Parr Instruments wurde mit Hilfe von Synthesegas (CO/H₂ = 1:1 (Vol-%)) Raffinat 3 hydroformyliert. Raffinat 3 ist ein Gemisch aus: ca. 26% 1-Buten, 32% trans-2-Buten, 17% cis-2-Buten, 25% n-Butan und Spuren an Iso-Butan und Neo-Pentan. Als Precursor wurden Rh(acac)(CO)₂ in Toluol vorgelegt. Der Ligand wurde im molaren Überschüssen von 4:1 relativ zum Rhodium verwendet. Als Stabilisator wurde Tinuvin 770DF im molaren Verhältnis ca. 1:1 zum Liganden eingesetzt. Zusätzlich wurden als GC-Standard ca. 0.5 g Tetraisopropylbenzol (TIPB) zugefügt. Ca. 9 g Edukt wurden nach Erreichen der vorgesehenen Reaktionstemperatur zudosiert.

Während der Reaktion wurde der Druck über eine Synthesegasregelung mit Massedurchflussmesser konstant gehalten. Die Rührerdrehzahl betrug 1200 min⁻¹. Proben wurden aus der Reaktionsmischung nach 5 Stunden gezogen. Die Ergebnisse der Versuche sind in Tabelle 1 zusammengefasst.

### Versuchsbeschreibung - speziell

In einem 100 ml-Autoklaven der Fa. Parr Instruments wurde bei 128 °C und 43 bar Synthesegasdruck 8.8 g Raffinat 3 hydroformyliert. Als Precursor wurden Rh(acac)(CO)₂ (100 ppm Rh) in 45 g Toluol vorgelegt. Als Ligand wurden 0.107 g Ligand in der Katalysatoransatzlösung eingesetzt. Als organisches Amin wurden 0.057 g Tinuvin 770DF, sowie 0.451 g TIPB als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Proben wurden aus der Reaktionsmischung nach 5 Stunden gezogen.

In Tabelle 1 sind die Hydroformylierungsergebnisse von Raffinat 3 bei 43 bar Synthesgasdruck und 128 °C dargestellt.

**Tabelle 1:**

| Eintrag | Ligand | Ausbeute Aldehyd in [%] | Regioselektivität n-Pentanal in % |
|---|---|---|---|
| 1 | **1*** | 82 | 43 |
| 2 | **2** | 54 | 99 |

| | | | |
|---|---|---|---|
| ∗ erfindungsgemäße Verbindung | | | |

### Definition der Selektivität:

Bei der Hydroformylierung gibt es die n/iso-Selektivitäte: das Verhältnis von linearem Aldehyd (= n) zu verzweigtem Aldehyd (= iso). Hierbei bedeutet die Regioselektivität bezüglich des n-Aldehyden, dass diese Menge an linearem Produkt gebildet wurde. Die restlichen Prozente entsprechen dann dem verzweigten Isomer. Bei einer Regioselektivität von 50% entstehen also n-Aldehyd und iso-Aldehyd zu gleichen Teilen.

Mit der erfindungsgemäßen Verbindung (**1**) konnte die Regioselektivität im Vergleich zum Vergleichsliganden (**2**) deutlich der 50%-Marke angenähert werden, welche für ein ausgeglichenes Verhältnis von n-Aldehyd und iso-Aldehyd steht.

Die durchgeführten Versuche belegen, dass die gestellten Aufgaben durch die erfindungsgemäße Verbindung (**1**) gelöst werden.

## Patentansprüche

1. Verbindung der Formel (**I**): 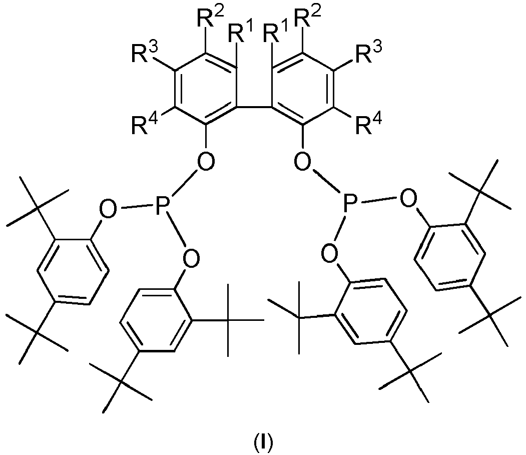 wobei
R¹, R², R³, R⁴ ausgewählt sind aus: -H, -(C₁-C₁₂)-Alkyl, -O-(C₁-C₁₂)-Alkyl,
wobei die genannten Alkylgruppen wie folgt substituiert sein können:
substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen; die Substituenten sind unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl,
wobei mindestens einer der Reste R¹, R², R³, R⁴ nicht für -H steht.

2. Verbindung nach Anspruch 1,
wobei R² und R⁴ ausgewählt sind aus: -(C₁-C₁₂)-Alkyl, -O-(C₁-C₁₂)-Alkyl.

3. Verbindung nach einem der Ansprüche 1 oder 2,
wobei R² für -O-(C₁-C₁₂)-Alkyl steht.

4. Verbindung nach einem der Ansprüche 1 bis 3,
wobei R⁴ für -(C₁-C₁₂)-Alkyl steht.

5. Verbindung nach einem der Ansprüche 1 bis 4,
wobei R² ausgewählt ist aus: -Me, -tBu, -OMe.

6. Verbindung nach einem der Ansprüche 1 bis 5,
wobei R² für -OMe steht.

7. Verbindung nach einem der Ansprüche 1 bis 6,
wobei R⁴ ausgewählt ist aus: -Me, -tBu.

8. Verbindung nach einem der Ansprüche 1 bis 7,
wobei R⁴ für -tBu steht.

9. Verbindung nach einem der Ansprüche 1 bis 8,
gemäß der Formel (**1**):

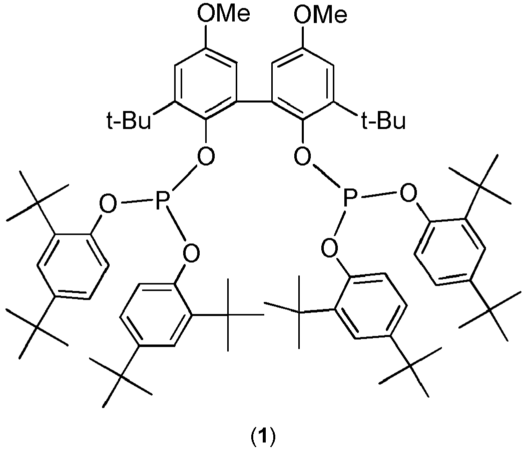

10. Komplex gemäß der Formel (**II**): 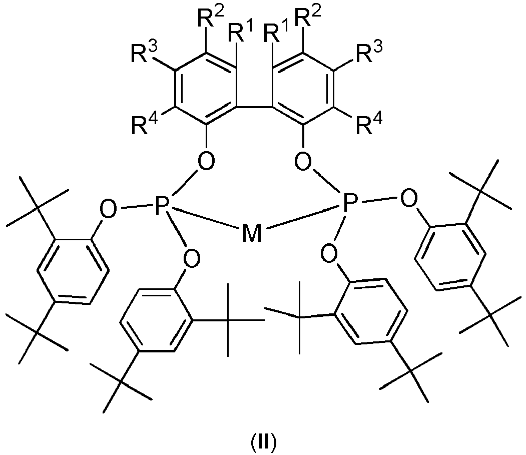 wobei
R¹, R², R³, R⁴ ausgewählt sind aus: -H, -(C₁-C₁₂)-Alkyl, -O-(C₁-C₁₂)-Alkyl,
wobei die genannten Alkylgruppen wie folgt substituiert sein können:
substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen; die Substituenten sind unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl;
wobei mindestens einer der Reste R¹, R², R³, R⁴ nicht für -H steht,
und M ausgewählt ist aus: Rh, Ru, Co, Ir.

11. Komplex gemäß der Formel (**III**): 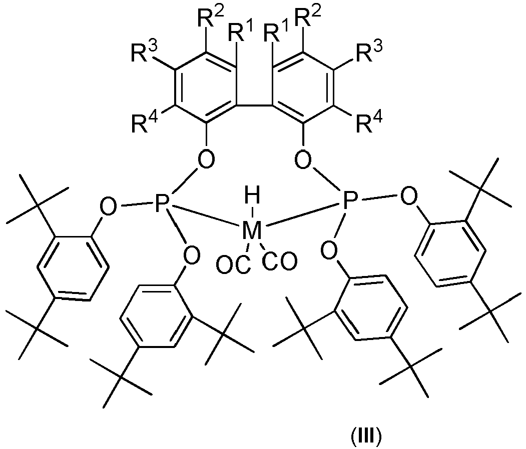 wobei
R¹, R², R³, R⁴ ausgewählt sind aus: -H, -(C₁-C₁₂)-Alkyl, -O-(C₁-C₁₂)-Alkyl,
wobei die genannten Alkylgruppen wie folgt substituiert sein können:
substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen; die Substituenten sind unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl;
wobei mindestens einer der Reste R¹, R², R³, R⁴ nicht für -H steht,
und M ausgewählt ist aus: Rh, Ru, Co, Ir.

12. Komplex nach Anspruch 10 oder 11,
wobei M = Rh ist.

13. Verwendung einer Verbindung nach Anspruch 1 bis 9,
in einem Ligand-Metall-Komplex zur Katalyse einer Hydroformylierungsreaktion.

14. Verfahren umfassend die Verfahrensschritte:
a) Vorlegen eines Olefins,
b) Zugabe eines Komplexes nach einem der Ansprüche 10 oder 11,
oder einer Verbindung nach einem der Ansprüche 1 bis 9 und einer Substanz, welche ein Metall ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

## Claims

1. Compound of the formula (I): 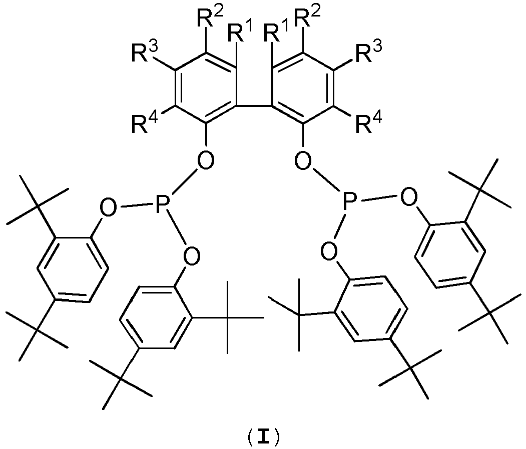 where
R¹, R², R³, R⁴ are selected from: -H, -(C₁-C₁₂)-alkyl,-O-(C₁-C₁₂)-alkyl,
wherein the alkyl groups mentioned may be substituted as follows:
substituted -(C₁-C₁₂)-alkyl groups and substituted -(C₁-C₁₂)-alkoxy groups, depending on their chain length, may have one or more substituents; the substituents are mutually independently selected from -(C₃-C₁₂)-cycloalkyl, -(C₃-C₁₂)-heterocycloalkyl, -(C₆-C₂₀)-aryl, fluorine, chlorine, cyano, formyl, acyl or alkoxycarbonyl, where at least one of the R¹, R², R³, R⁴ radicals is not -H.

2. Compound according to Claim 1,
where R² and R⁴ are selected from: -(C₁-C₁₂)-alkyl, -O-(C₁-C₁₂)-alkyl.

3. Compound according to either of Claims 1 and 2,
where R² is -O-(C₁-C₁₂)-alkyl.

4. Compound according to any of Claims 1 to 3,
where R⁴ is -(C₁-C₁₂)-alkyl.

5. Compound according to any of Claims 1 to 4,
where R² is selected from: -Me, -tBu, -OMe.

6. Compound according to any of Claims 1 to 5,
where R² is -OMe.

7. Compound according to any of Claims 1 to 6,
where R⁴ is selected from: -Me, -tBu.

8. Compound according to any of Claims 1 to 7,
where R⁴ is -tBu.

9. Compound according to any of Claims 1 to 8, according to the formula (1):

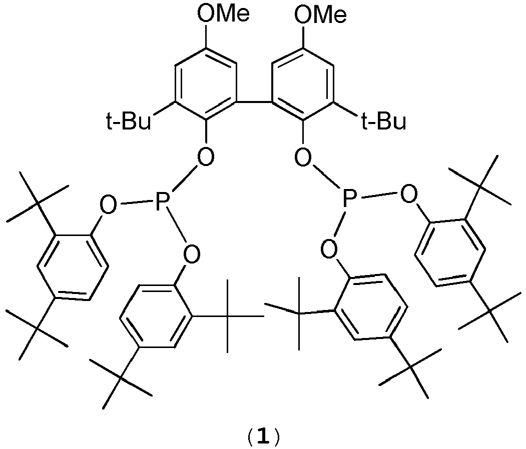

10. Complex according to the formula (II): 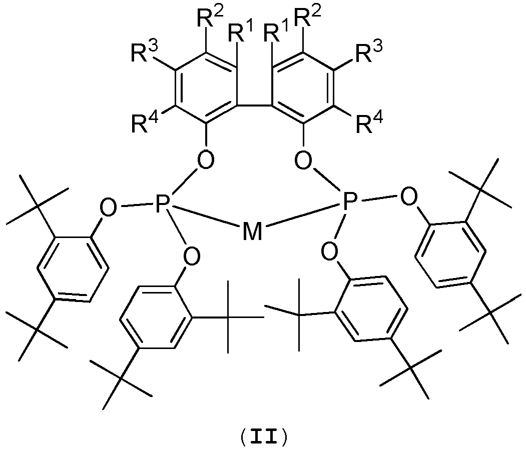 where
R¹, R², R³, R⁴ are selected from: -H, -(C₁-C₁₂)-alkyl,-O-(C₁-C₁₂)-alkyl,
wherein the alkyl groups mentioned may be substituted as follows:
substituted -(C₁-C₁₂)-alkyl groups and substituted -(C₁-C₁₂)-alkoxy groups, depending on their chain length, may have one or more substituents; the substituents are mutually independently selected from -(C₃-C₁₂)-cycloalkyl, -(C₃-C₁₂)-heterocycloalkyl, -(C₆-C₂₀)-aryl, fluorine, chlorine, cyano, formyl, acyl or alkoxycarbonyl;
where at least one of the R¹, R², R³, R⁴ radicals is not -H,
and M is selected from: Rh, Ru, Co, Ir.

11. Complex according to the formula (**III**): 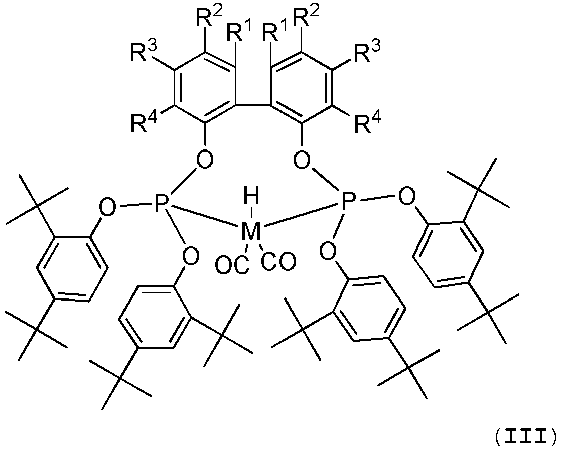 where
R¹, R², R³, R⁴ are selected from: -H, -(C₁-C₁₂)-alkyl,-O-(C₁-C₁₂)-alkyl,
wherein the alkyl groups mentioned may be substituted as follows:
substituted -(C₁-C₁₂)-alkyl groups and substituted -(C₁-C₁₂)-alkoxy groups, depending on their chain length, may have one or more substituents; the substituents are mutually independently selected from -(C₃-C₁₂)-cycloalkyl, -(C₃-C₁₂)-heterocycloalkyl, -(C₆-C₂₀)-aryl, fluorine, chlorine, cyano, formyl, acyl or alkoxycarbonyl;
where at least one of the R¹, R², R³, R⁴ radicals is not -H,
and M is selected from: Rh, Ru, Co, Ir.

12. Complex according to Claim 10 or 11,
where M = Rh.

13. Use of a compound according to Claims 1 to 9,
in a ligand-metal complex for catalysis of a hydroformylation reaction.

14. Process comprising the following process steps:
a) initially charging an olefin,
b) adding a complex according to either of Claims 10 and 11,
or a compound according to any of Claims 1 to 9 and a substance including a metal selected from: Rh, Ru, Co, Ir,
c) feeding in H₂ and CO,
d) heating the reaction mixture, wherein the olefin is converted to an aldehyde.

## Revendications

1. Composé de formule (I) : 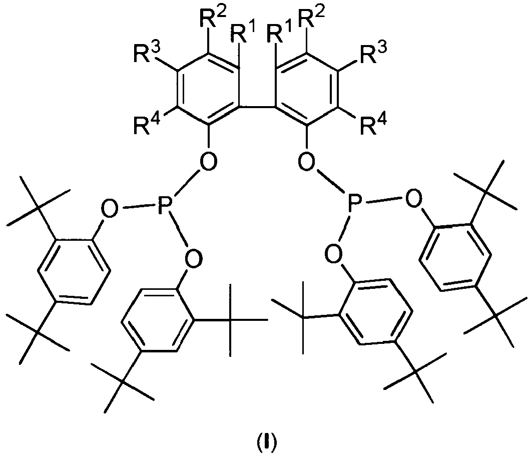 dans laquelle
R¹, R², R³, R⁴ sont choisis parmi : -H, -(C₁-C₁₂)-alkyle, -O-(C₁-C₁₂)-alkyle, les groupes alkyle mentionnés pouvant être substitués comme suit : les groupes -(C₁-C₁₂)-alkyle substitués et les groupes -(C₁-C₁₂)-alcoxy substitués peuvent présenter un ou plusieurs substituants, en fonction de leur longueur de chaîne ; les substituants sont choisis, indépendamment les uns des autres, parmi-(C₃-C₁₂)-cycloalkyle, -(C₃-C₁₂)-hétérocycloalkyle, -(C₆-C₂₀)-aryle, fluor, chlore, cyano, formyle, acyle ou alcoxycarbonyle, au moins un des radicaux R¹, R², R³, R⁴ ne représentant pas -H.

2. Composé selon la revendication 1, R² et R⁴ étant choisis parmi : -(C₁-C₁₂)-alkyle, -O-(C₁-C₁₂)-alkyle.

3. Composé selon l'une quelconque des revendications 1 ou 2, R² représentant -O-(C₁-C₁₂)-alkyle.

4. Composé selon l'une quelconque des revendications 1 à 3, R⁴ représentant -(C₁-C₁₂)-alkyle.

5. Composé selon l'une quelconque des revendications 1 à 4, R² étant choisi parmi : -Me, -tBu, -OMe.

6. Composé selon l'une quelconque des revendications 1 à 5, R² représentant -OMe.

7. Composé selon l'une quelconque des revendications 1 à 6, R⁴ étant choisi parmi : -Me, -tBu.

8. Composé selon l'une quelconque des revendications 1 à 7, R⁴ représentant -tBu.

9. Composé selon l'une quelconque des revendications 1 à 8, selon la formule (1) :

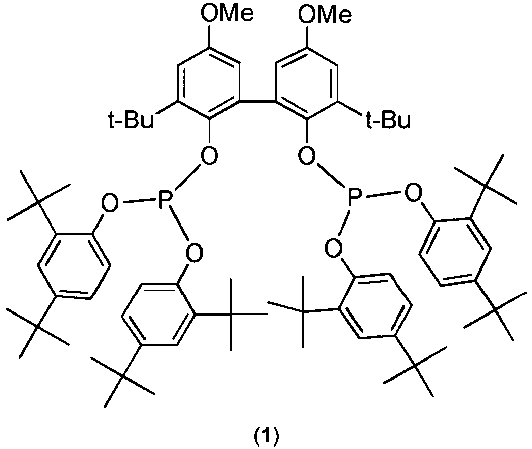

10. Complexe selon la formule (II) : 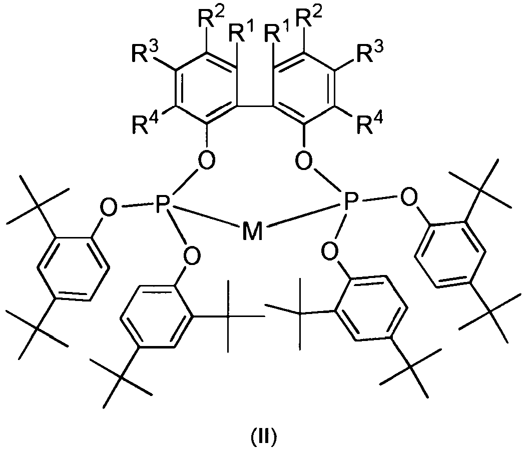 dans laquelle R¹, R², R³, R⁴ sont choisis parmi : -H,-(C₁-C₁₂)-alkyle, -O-(C₁-C₁₂)-alkyle, les groupes alkyle mentionnés pouvant être substitués comme suit : les groupes -(C₁-C₁₂)-alkyle substitués et les groupes -(C₁-C₁₂)-alcoxy substitués peuvent présenter un ou plusieurs substituants, en fonction de leur longueur de chaîne ; les substituants sont choisis, indépendamment les uns des autres, parmi -(C₃-C₁₂)-cycloalkyle, -(C₃-C₁₂)-hétérocycloalkyle, -(C₆-C₂₀)-aryle, fluor, chlore, cyano, formyle, acyle ou alcoxycarbonyle, au moins un des radicaux R¹, R², R³, R⁴ ne représentant pas -H et M est choisi parmi Rh, Ru, Co, Ir.

11. Complexe selon la formule (III) : 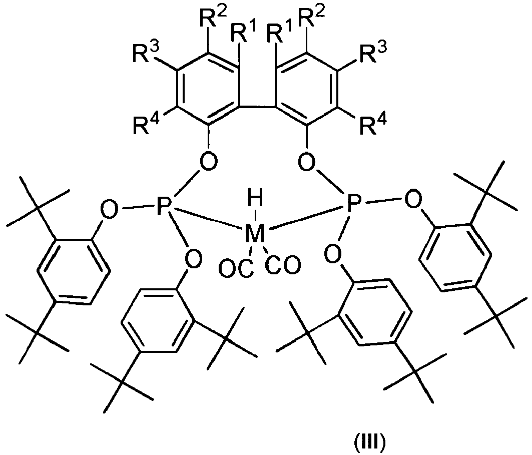 dans laquelle R¹, R², R³, R⁴ sont choisis parmi : -H,-(C₁-C₁₂)-alkyle, -O-(C₁-C₁₂)-alkyle, les groupes alkyle mentionnés pouvant être substitués comme suit : les groupes -(C₁-C₁₂)-alkyle substitués et les groupes -(C₁-C₁₂)-alcoxy substitués peuvent présenter un ou plusieurs substituants, en fonction de leur longueur de chaîne ; les substituants sont choisis, indépendamment les uns des autres, parmi -(C₃-C₁₂)-cycloalkyle, -(C₃-C₁₂)-hétérocycloalkyle, -(C₆-C₂₀)-aryle, fluor, chlore, cyano, formyle, acyle ou alcoxycarbonyle, au moins un des radicaux R¹, R², R³, R⁴ ne représentant pas -H et M est choisi parmi Rh, Ru, Co, Ir.

12. Complexe selon la revendication 10 ou 11, M = Rh.

13. Utilisation d'un composé selon la revendication 1 à 9 dans un complexe ligand-métal pour la catalyse d'une réaction d'hydroformylation.

14. Procédé comprenant les étapes de procédé :
a) disposition préalable d'une oléfine,
b) addition d'un complexe selon l'une quelconque des revendications 10 ou 11,
ou d'un composé selon l'une quelconque des revendications 1 à 9 et d'une substance qui présente un métal choisi parmi : Rh, Ru, Co, Ir,
c) introduction de H₂ et de CO,
d) chauffage du mélange réactionnel, l'oléfine étant transformée en aldéhyde.
